# EUROPÄISCHE PATENTSCHRIFT

(11) **EP 1 890 600 B1**
(45) Veröffentlichungstag und Bekanntmachung des Hinweises auf die Patenterteilung: **07.07.2010**
(21) Anmeldenummer: 06754008.8
(22) Anmeldetag: 31.05.2006
(51) Int. Cl.: A61B 5/103, A61B 19/00

(54) **CHIRURGISCHES NAVIGATIONSSYSTEM ZUR HERSTELLUNG EINER AUFNAMEVERTIEFUNG FÜR EINE HÜFTGELENKPFANNE**
SURGICAL NAVIGATION SYSTEM FOR CREATING A RECESS TO RECEIVE AN ACETABULUM
SYSTEME DE NAVIGATION CHIRURGICAL POUR REALISER UN EVIDEMENT DE RECEPTION POUR UN COTYLE

(30) Priorität: 15.06.2005 DE 102005028831
(43) Veröffentlichungstag der Anmeldung: 27.02.2008
(73) Patentinhaber: Aesculap AG, 78532 Tuttlingen (DE)
(72) Erfinder: MOLLARD, Benoit, F-38130 Echirolles (FR); LEITNER, François, 38410 Uriage (FR); KAMMERZELL, Sergej, 78234 Engen (DE); FRIEDRICH, Dirk, 78532 Tuttlingen (DE)
(74) Vertreter: Hoeger, Stellrecht & Partner Patentanwälte
(86) Internationale Anmeldenummer: PCT/EP2006/005181
(87) Internationale Veröffentlichungsnummer: WO 2006/133806

(56) Entgegenhaltungen:
- WO-A-2004/030556
- DE-U1-202005 009 777
- US-A1- 2002 077 540

## Beschreibung

Die Erfindung betrifft ein chirurgisches Navigationssystem für ein navigiertes Werkzeug zur Herstellung einer Aufnahmevertiefung für eine Hüftgelenkpfanne in einem navigierten Beckenknochen mit einem navigierten Tastinstrument und mit einer Datenverarbeitungseinrichtung zur Bearbeitung der Lagedaten der navigierten Teile.

Bei der Implantation eines künstlichen Hüftgelenkes wird eine künstliche Hüftgelenkpfanne in den Beckenknochen eingesetzt, dazu muß an der anatomisch vorgegebenen Stelle in anatomisch vorgegebener Orientierung eine Vertiefung in den Beckenknochen eingearbeitet werden, die die Hüftgelenkpfanne aufnimmt. Diese Vertiefungen sind normalerweise halbkugelförmig ausgebildet und werden mittels eines halbkugelförmigen Fräswerkzeuges erzeugt. Um die genaue Lage - also die Orientierung und die Position - der Aufnahmevertiefung bestimmen und einhalten zu können, ist es bekannt, den Beckenknochen sowie das die Vertiefung erzeugende Werkzeug mittels eines chirurgischen Navigationssystems zu navigieren. Durch ein solches Navigationssystem kann die relative räumliche Zuordnung des Werkzeuges und des Beckenknochens bestimmt und überwacht werden.

Bei dem Ersetzen einer defekten Hüftgelenkpfanne durch eine künstliche Hüftgelenkpfanne wird normalerweise die Hüftgelenkpfanne im wesentlichen am selben Ort implantiert, an dem vorher die natürliche Hüftgelenkpfanne angeordnet war (WO 2004/030556 A2), diese Position wird nachfolgend als primäres Acetabulum bezeichnet.

Es gibt aber auch Fälle, in denen sich die natürliche Hüftgelenkpfanne nicht an der anatomisch gewünschten Position befindet, sei es durch einen Geburtsfehler, sei es durch eine Verschiebung der natürlichen Gelenkpfanne aus der Position des primären Acetabulums heraus, üblicherweise nach oben, das heißt in cranialer Richtung. In diesen Fällen ist es außerordentlich schwierig, bei der Implantation einer künstlichen Gelenkpfanne, die an der anatomisch normalen Position, also in der Position des primären Acetabulums, eingesetzt werden soll, diese Lage am Beckenknochen zu bestimmen, so daß dann das Werkzeug in der gewünschten Weise geführt werden kann.

Es ist Aufgabe der Erfindung, ein chirurgisches Navigationssystem der gattungsgemäßen Art so auszubilden, dass dem Chirurgen für die richtige Lage und Orientierung des Werkzeuges relativ zum Beckenknochen eine Hilfestellung gegeben wird, so daß die Aufnahmevertiefung für die Hüftgelenkpfanne an der gewünschten anatomischen Stelle und in der gewünschten Orientierung in den Beckenknochen eingearbeitet werden kann, auch wenn die natürliche Hüftgelenkpfanne sich vorher nicht in dieser gewünschten anatomischen Stellung befunden hat.

Diese Aufgabe wird bei einem chirurgischen Navigationssystem der eingangs beschriebenen Art erfindungsgemäß dadurch gelöst, dass die Datenverarbeitungseinrichtung derart programmiert ist, dass sie aus den mit Hilfe des navigierten Tastinstrumentes bestimmten Lagedaten eines tear drop points (Köhlersche Tränenfigur) am Beckenknochen, aus den Lagedaten der Beckeneingangsebene und aus den Lagedaten der beiden Spinae illiacae anteriores superiores des Beckenknochens eine senkrecht auf der Beckeneingangsebene stehende, parallel zu einer die beiden Spinae illiacae anteriores superiores verbindenden Linie und durch den getasteten tear drop point verlaufende tear drop Ebene bestimmt, dass sie in einer vorgegebenen Höhe oberhalb der tear drop Ebene in einer vorgegebenen Position in anterior-posteriorer Richtung und in einem vorgegebenen Abstand von der Außenfläche des Beckenknochens in lateral-medialer Richtung einen Bezugspunkt bestimmt, dass sie unter Verwendung der Lagedaten eines gegenüber dem primären Acetabulum in kranialer Richtung verschobenen sekundären Acetabulums die Höhe des Bezugspunktes oberhalb der tear drop Ebene derart berechnet, dass ein maximaler Wert des Abstandes zwischen der Höhe des Bezugspunktes und dem sekundären Acetabulum nicht überschritten wird, und dass sie die Lage des Werkzeuges relativ zu dem Beckenknochen und relativ zu dem Bezugspunkt berechnet.

An Hand von relativ wenigen markanten Punkten des Beckenknochens und aufgrund gewisser anatomischer Annahmen gelingt es auf diese Weise, die Lage des Werkzeuges, mit dem die Aufnahmevertiefung hergestellt wird, so zu wählen, daß die Aufnahmevertiefung in der gewünschten Position und Orientierung am Beckenknochen ausgebildet werden kann.

Ausgehend von der Beckeneingangsebene des Beckenknochens, die durch die beiden Spinae illiacae anteriores superiores einerseits und die Symphysis pubica andererseits aufgespannt wird, sowie durch die Bestimmung der Lage der tear drop Punkte gelingt es, die tear drop Ebene zu bestimmen, also eine horizontale Ebene des Beckens, die durch den tear drop hindurchgeht und eine Bezugsebene bildet, von der ausgehend Lage und Orientierung der Aufnahmevertiefung bestimmt werden können.

Dazu wird ein Bezugspunkt bestimmt, der sich in bestimmter Höhe oberhalb dieser tear drop Ebene befindet und dessen Lage in anterior-posteriorer Richtung durch anatomische Annahmen ausgewählt wird.

Schließlich wird noch der Abstand dieses Bezugspunktes von der Außenfläche des Beckenknochens bestimmt, und zwar in lateral-medialer Richtung. Auch hier werden anatomische Annahmen verwertet, um diesen Bezugspunkt zu bestimmen, er kann beispielsweise innerhalb des Beckenknochens liegen oder in einem anderen, besonders bevorzugten Fall mit der Rückseite des Beckenknochens im Bereich des Acetabulums zusammenfallen.

Sobald dieser Bezugspunkt bekannt ist, kann das Navigationssystem die relative Lage des Werkzeuges zu dem Beckenknochen und insbesondere zu dem Bezugspunkt laufend überwachen und damit dem Chirurgen eine Kontrollmöglichkeit an die Hand geben für die Führung des Werkzeuges und die dadurch erzielbare, anatomisch richtige Lage der Aufnahmevertiefung im Beckenknochen. Insbesondere kann die Gefahr herabgesetzt werden, daß die Aufnahmevertiefung zu tief in den Beckenknochen eingearbeitet wird, so daß im Bereich des Acetabulums eine zu dünne Knochenwand stehen bleibt oder gar eine unerwünschte Fensterung des Knochens auftritt.

Es ist vorteilhaft, wenn man zur Bestimmung der Lage der Beckeneingangsebene mittels des navigierten Tastinstrumentes die beiden Spinae illiacae anteriores superiores des Beckenknochens sowie die Symphysis pubica anfährt und aus den Lagedaten dieser drei Punkte die Beckeneingangsebene berechnet.

Insofern kann also ebenfalls auf anatomische Gegebenheiten des Beckenknochens zurückgegriffen werden, diese Abtastung der drei genannten Punkte kann extrakorporal erfolgen.

Günstig ist es, wenn man die Höhe des Bezugspunktes oberhalb der tear drop Ebene in Abhängigkeit von der Dimensionierung des Werkzeuges und damit der gewünschten Größe der Aufnahmevertiefung so auswählt, daß der untere Rand der Aufnahmevertiefung etwa in der tear drop Ebene liegt.

Die Aufnahmevertiefung wird in der Regel mit einem halbkugelförmigen Fräser eingearbeitet, so daß die halbkugelige Aufnahmevertiefung durch diese Dimensionierung in ihrer Höhe wohl definiert ist.

Bei einer Verschiebung des sekundären Acetabulums, in dem der Femurknochen vor der Operation im Beckenknochen gelagert ist, in cranialer Richtung, ergibt sich für den Patienten präoperativ eine Verkürzung des Beines gegenüber einer normalen anatomischen Lagerung im primären Acetabulum, in dem ein Femur bei anatomisch richtiger Lagerung gelagert wäre. Wenn man die neue Hüftgelenkpfanne im Bereich des primären Acetabulums implantiert, resultiert daraus zwangsläufig eine Verlängerung des Beines , die normalerweise in Kauf genommen wird. Allerdings hat sich herausgestellt, daß diese Verlängerung des Beines bis zu einer Größenordnung von etwa 25 mm unproblematisch ist, daß aber bei einer größeren Verlängerung, insbesondere einer Verlängerung über 40 mm hinaus, Probleme auftreten können, da die Muskeln und Nerven dann übermäßig gedehnt werden. Dies kann zu Schäden führen und sollte vermieden werden.

Daher ist gemäß einem bevorzugten Beispiel 2 vorgesehen, daß man die Höhe des Bezugspunktes oberhalb der tear drop Ebene derart wählt, daß ein maximaler Wert des Abstandes zwischen der Höhe des Bezugspunktes und der Lage eines gegenüber dem primären Acetabulum in cranialer Richtung verschobenen sekundären Acetabulums nicht überschritten wird. Dieser maximale Wert sollte bei maximal 40 mm, vorzugsweise bei 25 mm.

Es ist dabei vorteilhaft, wenn der maximale Wert zwischen einem zentralen Punkt des sekundären Acetabulums und dem Bezugspunkt bestimmt wird.

Gemäß eines bevorzugten Beispiels kann man zur Bestimmung des zentralen Punktes den navigierten Femurknochen präoperativ gegenüber dem navigierten Beckenknochen in dem sekundären Acetabulum bewegen, aus der Bewegung den Mittelpunkt der Bewegung des Femurknochens bestimmen und diesen Mittelpunkt als zentralen Punkt des sekundären Acetabulums verwenden.

Bei einem alternativen Beispiel 2 kann man zur Bestimmung des zentralen Punktes ein navigiertes Tastinstrument an die Lagerfläche des sekundären Acetabulums anlegen und dadurch die Lage des sekundären Acetabulums bestimmen, wobei man dann aus den Lagedaten des sekundären Acetabulums deren Mittelpunkt als zentralen Punkt des sekundären Acetabulums bestimmt. Ein solches Tastinstrument kann die Lagerfläche entweder punktförmig abtasten, es ist aber auch möglich, eine etwa halbkugelige Anlagefläche an dem Tastinstrument in das sekundäre Acetabulum einzulegen und dadurch die Lagedaten der Lagerfläche des sekundären Acetabulums zu bestimmen.

Es kann vorgesehen werden, daß man die Position des Bezugspunktes in anterior-posteriorer Richtung so auswählt, daß er etwa in der Mitte der ursprünglichen Aufnahmevertiefung liegt.

Es ist wünschenswert, die Aufnahmevertiefung so zu plazieren, daß der dorsale Randbereich des Beckenknochens in Höhe der Aufnahmevertiefung vollständig erhalten bleibt, also nicht von dem die Aufnahmevertiefung erzeugenden Werkzeug entfernt wird, da eine Zerstörung oder ein Abtragen dieses dorsalen Randes die Fixierung der Hüftgelenkpfanne in der Aufnahmevertiefung beeinträchtigen könnte. Dieser dorsale Rand ist ein Knochenvorsprung des Beckenknochens und hat die Form eines Halbmondes.

Um dieses Ziel zu erreichen, kann gemäß eines bevorzugten Beispiels vorgesehen sein, daß man mittels eines navigierten Tasters die Lagedaten eines oder mehrerer Punkte des dorsalen Randes des Beckenknochens im Bereich des primären Acetabulums bestimmt und die Aufnahmevertiefung so positioniert, daß der Abstand des Randes der Aufnahmevertiefung von der Lage des Meßpunktes oder der Meßpunkte des dorsalen Randes einen bestimmten Minimalwert nicht unterschreitet. Wenn man mit dem Taster nur einen Punkt des dorsalen Randes erfaßt, wird der Abstand zwischen diesem Punkt und dem Rand der Aufnahmevertiefung bestimmt, wenn man aber gemäß eines bevorzugten Beispiels mehrere Meßpunkte bestimmt, dann kann vorgesehen werden, daß man aus den Lagedaten dieser mehreren Meßpunkte des dorsalen Randes des Beckenknochens denjenigen auswählt, der am weitesten in ventraler Richtung angeordnet ist, und dann ist es vorteilhaft, wenn man den bestimmten Minimalwert aus den Lagedaten dieses ausgewählten Meßpunktes bestimmt. Auf diese Weise ist sichergestellt, daß auch bei einer relativ ungenauen Erfassung der Lagedaten des dorsalen Randes ein maximaler Abstand von dem dorsalen Rand eingehalten und dieser bei der Einbringung der Aufnahmevertiefung nicht beschädigt wird.

Man kann bei der Bestimmung der Position der Aufnahmevertiefung und bei der Führung des Werkzeuges, mit dem diese Aufnahmevertiefung erzeugt wird, den minimalen Abstand zwischen der Außenfläche des Werkzeug oder dem Mittelpunkt des Werkzeuges einerseits und dem ausgewählten Meßpunkt andererseits bestimmen, es ist aber gemäß einer bevorzugten Ausführungsform auch vorgesehen, daß man eine durch einen ausgewählten Meßpunkt des dorsalen Randes hindurchgehende dorsale Frontalebene bestimmt und den bestimmten Minimalwert zwischen dem Rand der Aufnahmevertiefung und dieser dorsalen Frontalebene bestimmt. Diese dorsale Frontalebene verläuft im wesentlichen parallel zur Beckeneingangsebene und durch den ausgewählten Meßpunkt hindurch. Wenn die Außenfläche des Werkzeuges oder ihr Mittelpunkt einen bestimmten Abstand gegenüber dieser dorsalen Frontalebene einhalten, ist ebenfalls sichergestellt, daß eine Beschädigung des dorsalen Randes des Beckenknochens nicht erfolgen kann.

Besonders vorteilhaft ist es, wenn man den Abstand des Bezugspunktes von der Außenfläche des Beckenknochens aus den Lagedaten eines navigierten Tastinstrumentes bestimmt, welches durch eine künstliche Öffnung im Bereich des primären Acetabulums des Beckenknochens in medialer Richtung durch den Beckenknochen bis zur Rückseite des Beckenknochens hindurchgesteckt wird. Es wird also vom primären Acetabulum aus eine Bohrung oder eine Durchbrechung durch den Beckenknochen hindurchgearbeitet, so daß das Tastinstrument bis an die Rückseite des Beckenknochens vorgeschoben werden kann und damit die Tiefe bestimmen kann, die innerhalb des Knochens maximal zur Verfügung steht, um die Aufnahmevertiefung einzuarbeiten.

Besonders günstig ist es dabei, wenn das Tastinstrument zur Bestimmung des Bezugspunktes von der Außenfläche des Beckenknochens mittels einer seitlich von einem Schaft abstehenden Anlagefläche an die Rückseite des Beckenknochens angelegt wird, beispielsweise kann diese Anlagefläche durch einen seitlich abstehenden Haken des Schaftes gebildet werden. Ein solches Tastinstrument kann durch die Öffnung im Beckenknochen hindurchgeschoben und dann soweit zurückgezogen werden, bis die Anlagefläche an der Rückseite des Bekkenknochens anliegt, dadurch läßt sich die Dicke des Beckenknochens in diesem Bereich bestimmen, und diese Position wird zur Bestimmung des Bezugspunktes herangezogen.

Bei der Einarbeitung der Aufnahmevertiefung kann gemäß eines ersten bevorzugten Beispiels vorgesehen sein, daß man das Werkzeug so führt, daß der kleinste Abstand seiner der Knochenbearbeitung dienenden Außenfläche von dem Bezugspunkt einen bestimmten Wert nicht unterschreitet. Es wird also beispielsweise dafür Sorge getragen, daß die kugelige Außenfläche des Fräsers sich dem Bezugspunkt nur so weit nähert, bis ein bestimmter Wert unterschritten wird.

Bei einem weiteren bevorzugten Beispiel kann vorgesehen sein, daß man das Werkzeug so führt, daß der kleinste Abstand seines Mittelpunktes von dem Bezugspunkt einen bestimmten Wert nicht unterschreitet. Dies ist insbesondere dann günstig, wenn halbkugelförmige Fräser mit unterschiedlichen Radien nacheinander benutzt werden, der Operateur kann dann darauf achten, daß die Mittelpunkte dieser Fräswerkzeuge sich dem Bezugspunkt nicht übermäßig nähern.

Statt den Abstand der Außenfläche des Werkzeuges oder seines Mittelpunktes relativ zu dem Bezugspunkt als kritische Größe zu wählen, könnte man auch den Abstand der Außenfläche oder des Mittelpunktes relativ zu einer durch diesen Bezugspunkt hindurchgehenden Sagittalebene wählen, in jedem Falle wird dafür Sorge getragen, daß die Abstände zu dem Bezugspunkt oder zu dieser Sagittalebene einen bestimmten Wert nicht unterschreiten, um die Tiefe der Aufnahmeöffnung auf ein zulässiges Maß zu begrenzen.

Die Datenverarbeitungseinrichtung ist so programmiert ist, daß sie aus den mittels des Tastinstrumentes bestimmten Lagedaten der beiden Spinae illiacae anteriores superiores und der Symphysis pubica des Beckenknochens die Beckeneingangsebene berechnet.

Bei einer bevorzugten Ausführungsform ist vorgesehen, daß die Datenverarbeitungseinrichtung so programmiert ist, daß sie die Höhe des Bezugspunktes oberhalb der tear drop Ebene in Abhängigkeit von der Dimensionierung des Werkzeuges und damit der gewünschten Größe der Aufnahmevertiefung so berechnet, daß der untere Rand der Aufnahmevertiefung etwa in der tear drop Ebene liegt.

Die Datenverarbeitungseinrichtung ist so programmiert, daß sie die Höhe des Bezugspunktes oberhalb der tear drop Ebene derart wählt, daß ein maximaler Wert des Abstandes zwischen der Höhe des Bezugspunktes und der Lage eines gegenüber dem primären Acetabulum in cranialer Richtung verschobenen sekundären Acetabulums nicht überschritten wird. Dieser maximale Wert kann bei 40 mm liegen, vorzugsweise bei 25 mm.

Die Datenverarbeitungseinrichtung kann dabei so programmiert sein, daß der maximale Wert zwischen einem zentralen Punkt des sekundären Acetabulums und dem Bezugspunkt bestimmt wird.

Dabei kann vorgesehen werden, daß die Datenverarbeitungseinrichtung so programmiert ist, daß sie aus den Lagedaten eines navigierten, gegenüber dem navigierten Beckenknochen in dem sekundären Acetabulum präoperativ bewegten Femurknochen den Mittelpunkt der Bewegung des Femurknochens bestimmt und diesen Mittelpunkt als zentralen Punkt des sekundären Acetabulums verwendet.

Alternativ kann vorgesehen sein, daß die Datenverarbeitungseinrichtung so programmiert ist, daß sie aus den Lagedaten eines navigierten Tastinstrumentes, welches an die Lagerfläche des sekundären Acetabulums angelegt ist, die Lage des sekundären Acetabulums bestimmt und daß sie aus den Lagedaten des sekundären Acetabulums deren Mittelpunkt als zentralen Punkt des sekundären Acetabulums bestimmt. Gemäß einer weiteren bevorzugten Ausführungsform ist vorgesehen, daß die Datenverarbeitungseinrichtung so programmiert ist, daß sie die Position des Bezugspunktes in anterior-posteriorer Richtung so auswählt, daß er etwa in der Mitte der Aufnahmevertiefung liegt.

Es ist weiterhin von Vorteil, wenn die Datenverarbeitungseinrichtung so programmiert ist, daß sie aus Lagedaten, die aus Anlage eines navigierten Tasters an einen oder mehrere Meßpunkte des dorsalen Randes des Beckenknochens im Bereich des primären Acetabulums bestimmt worden sind, eine Position der Aufnahmevertiefung derart bestimmt, daß der Abstand des Randes der Aufnahmevertiefung von den Lagedaten des Meßpunktes oder der Meßpunkte des dorsalen Randes einen bestimmten Minimalwert nicht unterschreitet.

Die Datenverarbeitungseinrichtung kann vorzugsweise so programmiert sein, daß sie aus den Lagedaten von mehreren Meßpunkten des dorsalen Randes des Beckenknochens denjenigen auswählt, der am weitesten in ventraler Richtung angeordnet ist, und daß sie den bestimmten Minimalwert aus den Lagedaten dieses ausgewählten Meßpunktes bestimmt.

Weiterhin kann die Datenverarbeitungseinrichtung so programmiert sein, daß sie eine durch einen Meßpunkt des dorsalen Randes hindurchgehende dorsale Frontalebene bestimmt und den bestimmten Minimalwert zwischen dem Rand der Aufnahmevertiefung oder dem Mittelpunkt der Aufnahmevertiefung und dieser dorsalen Frontalebene bestimmt.

Es kann weiterhin vorgesehen sein, daß die Datenverarbeitungseinrichtung so programmiert ist, daß sie den Abstand des Bezugspunktes von der Außenfläche des Beckenknochens aus den Lagedaten eines navigierten Tastinstrumentes bestimmt, welches durch eine künstliche Öffnung im Bereich des primären Acetabulums des Beckenknochens in medialer Richtung durch den Beckenknochen bis zur Rückseite des Beckenknochens hindurchgesteckt wird.

Es ist dabei günstig, wenn das Tastinstrument zur Bestimmung des Abstandes des Bezugspunktes von der Außenfläche des Beckenknochens eine seitlich von einem Schaft abstehende Anlagefläche zur Anlage an der Rückseite des Bekkenknochens aufweist.

Bei einer ersten bevorzugten Ausführungsform kann vorgesehen werden, daß die Datenverarbeitungseinrichtung so programmiert ist, daß sie den kleinsten Abstand der der Knochenbearbeitung dienenden Außenfläche des Werkzeuges von dem Bezugspunkt berechnet.

Statt des kleinsten Abstandes zwischen Außenfläche des Werkzeuges und Bezugspunkt kann auch der kleinste Abstand des Mittelpunktes des Werkzeuges und des Bezugspunktes bestimmt werden.

In anderen Fällen werden diese Abstände nicht gegenüber dem Bezugspunkt bestimmt, sondern gegenüber einer durch den Bezugspunkt hindurchgehenden Sagittalebene.

Es ist weiterhin vorteilhaft, wenn die Datenverarbeitungseinrichtung so programmiert ist, daß sie die relative Lage des Werkzeuges und des Bezugspunktes oder der durch den Bezugspunkt gehenden Sagittalebene auf einer Anzeige anzeigt.

Weiterhin ist es für den Operateur hilfreich, wenn die Datenverarbeitungseinrichtung so programmiert ist, daß sie ein Warnsignal erzeugt, sobald der bestimmte Wert unterschritten ist. Dadurch ist sichergestellt, daß das Werkzeug nicht zu tief in den Knochen eingearbeitet wird.

Die vorstehend beschriebenen Maßnahmen können besonders vorteilhaft in Kombination miteinander eingesetzt werden. Es ist aber auch möglich, bei der Vorbereitung der entsprechenden Operation nur einige dieser Schritte zu verwenden. Dies bezieht sich insbesondere auf die Bestimmung der Lage der Aufnahmevertiefung im Bezug auf die Höhe über der tear drop Ebene, im Bezug auf den Abstand der Aufnahmevertiefung relativ zum sekundären Acetabulum in cranialer Richtung, im Bezug auf den Abstand der Aufnahmevertiefung vom dorsalen Rand des Beckenknochens und im Hinblick auf die Bestimmung der Eintauchtiefe der Aufnahmevertiefung durch Positionierung eines Bezugspunktes im Beckenknochen oder an der Rückseite des Beckenknochens. Jede dieser Maßnahmen kann für sich vorteilhaft eingesetzt werden, es können auch mehrere oder alle dieser Maßnahmen gemeinsam Anwendung finden.

Die nachfolgende Beschreibung bevorzugter Ausführungsformen der Erfindung dient im Zusammenhang mit der Zeichnung der näheren Erläuterung. Es zeigen:

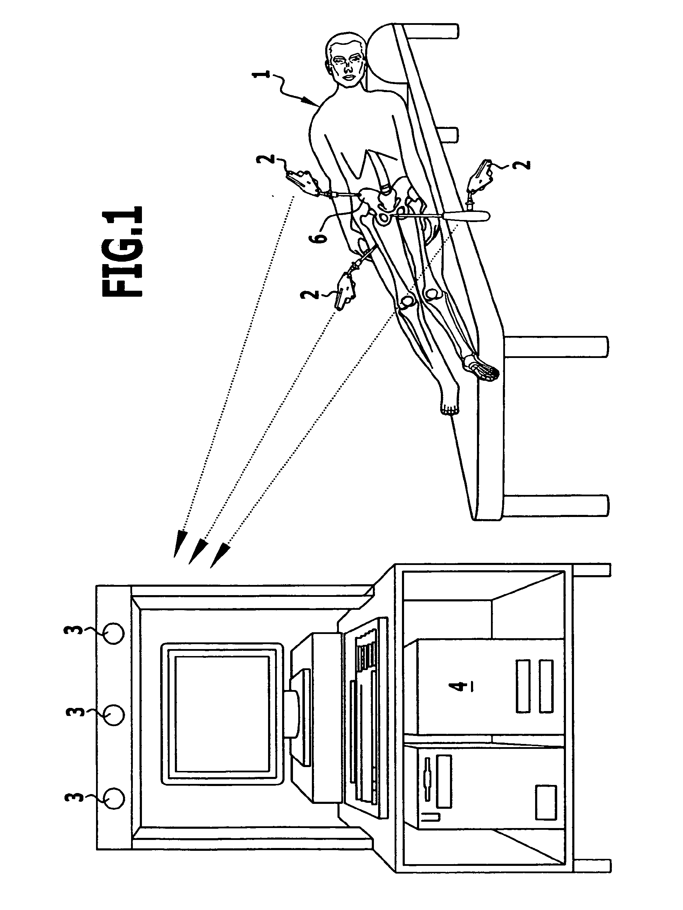

- Figur 1:: eine Ansicht eines Operationsraumes mit einem Navigationssystem und einem Patienten sowie chirurgischen Instrumenten, die mit Markierelementen für das Navigationssystem versehen sind;
- Figur 2:: eine schematische Darstellung eines mit einem Markierelement ver- sehenen Beckenknochens und der zur Bestimmung der Beckenein- gangsebene verwendeten Tastpunkte;
- Figur 3:: eine schematische Darstellung des Beckenknochens und eines navi- gierten Tastinstrumentes beim Abtasten eines tear drop Punkts;
- Figur 4:: eine schematische Seitenansicht des Beckenknochens mit einem durch eine künstliche Öffnung im Bereich des Acetabulums hin- durchgesteckten navigierten Tastinstrument ;
- Figur 5:: eine Seitenansicht eines Beckenknochens bei der Einarbeitung einer Aufnahmevertiefung mit Hilfe eines halbkugelförmigen Fräsers;
- Figur 6:: eine Lateralansicht eines Beckenknochens mit Meßpunkten am dor- salen Rand des Beckenknochens und mit Angaben zur Lage der tear drop Ebene, einer ventralen und einer dorsalen Frontalebene sowie der Lage eines primären und eines sekundären Acetabulums;
- Figur 7:: eine schematische Ansicht eines Beckenknochens mit einem in das sekundäre Acetabulum eingesetzten Tastinstrument zur Bestim- mung der Lagedaten des sekundären Acetabulums und
- Figur 8:: eine schematische Teilansicht des Beckenknochens mit einem pri- mären und einem sekundären Acetabulum und einer Angabe von deren Abstand in cranialer Richtung.

Für die Durchführung einer Hüftgelenks-Implantation an einem Patienten 1 wird ein Navigationssystem benutzt, welches die Position und Orientierung - zusammenfassend als Lage bezeichnet - von Markierelementen 2 im Raum bestimmen kann. Diese Markierelemente 2 können mit Körperteilen, Instrumenten etc. fest verbunden werden und erlauben somit, die Lage dieser Körperteile und Instrumente im Raum zu bestimmen. Die Bestimmung erfolgt dabei über im Abstand voneinander angeordnete, stationäre Sender 3, die gleichzeitig als Empfänger ausgebildet sind und die von ihnen ausgesandte Strahlung empfangen, welche an im Abstand zu einander angeordneten Reflektoren der Markierelemente 2 reflektiert wird.

Die Lagedaten der Markierelemente 2 und damit der starr mit ihnen verbundenen Körperteile und Instrumente werden in einer Datenverarbeitungseinrichtung 4 aufgenommen und weiterverarbeitet, auf einem von der Datenverarbeitungseinrichtung 4 gesteuerten Bildschirm 5 können diese Körperteile und Instrumente in ihrer relativen Lage zueinander dargestellt werden.

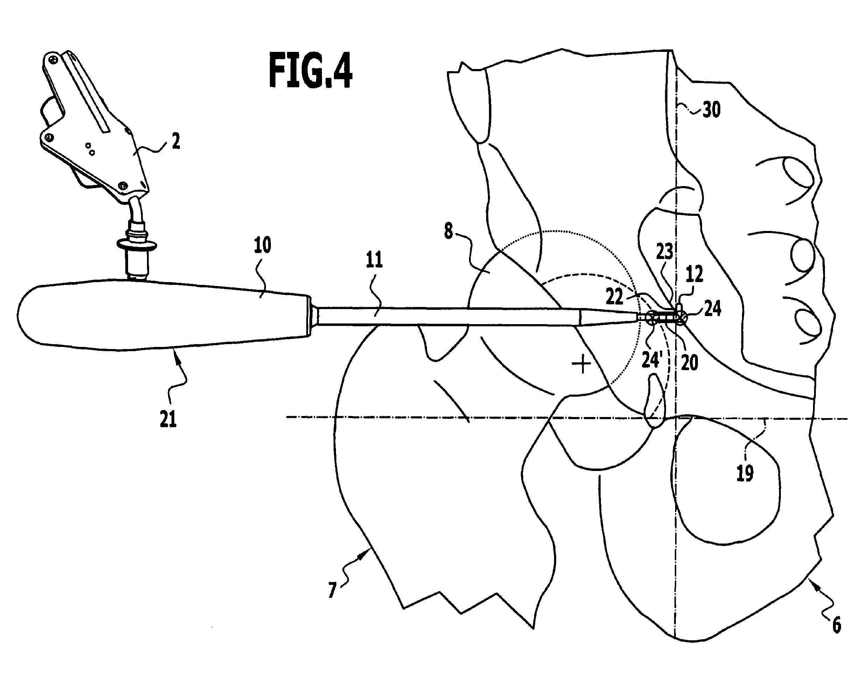

In Figur 4 ist schematisch ein Beckenknochen 6 dargestellt zusammen mit dem oberen Teil eines Femurknochens 7, der mit einem Femurkopf 8 in eine natürliche, in der Zeichnung nicht näher dargestellte Hüftgelenkpfanne eintaucht. In dem in Figur 4 dargestellten Beispiel ist diese Hüftgelenkpfanne gegenüber der normalen anatomischen Position nach oben - also in cranialer Richtung - verschoben, die Kontur dieser nach oben verschobenen Hüftgelenkpfanne wird durch eine Punktreihe gekennzeichnet.

Beim Ersatz des Hüftgelenkes soll das Hüftgelenk so nach unten - also in caudaler Richtung - verschoben werden, daß es die anatomisch richtige Lage im Beckenknochen einnimmt, diese anatomisch richtige Lage wird in Figur 4 durch eine gestrichelte Kreisbogenlinie angedeutet. Es ergibt sich daher die Aufgabe, diese anatomisch richtige Lage am Beckenknochen zu finden und zu bestimmen. Dazu kann nicht auf die natürliche Hüftgelenkschale zurückgegriffen werden, da diese aufgrund der krankhaften Veränderung von der anatomischen Idealposition in undefinierbarer Weise entfernt ist.

Es ist daher notwendig, durch bestimmte markante Punkte am Beckenknochen ein Referenzsystem aufzubauen, von dem ausgehend die Lage der die künstliche Hüftgelenkpfanne aufnehmenden Aufnahmevertiefung definiert werden kann.

Zu diesem Zweck wird einmal der Beckenknochen 6 mit einem Markierelement 2 starr verbunden, dies kann beispielsweise mittels einer Knochenschraube in den Beckenknochen eingeschraubt sein.

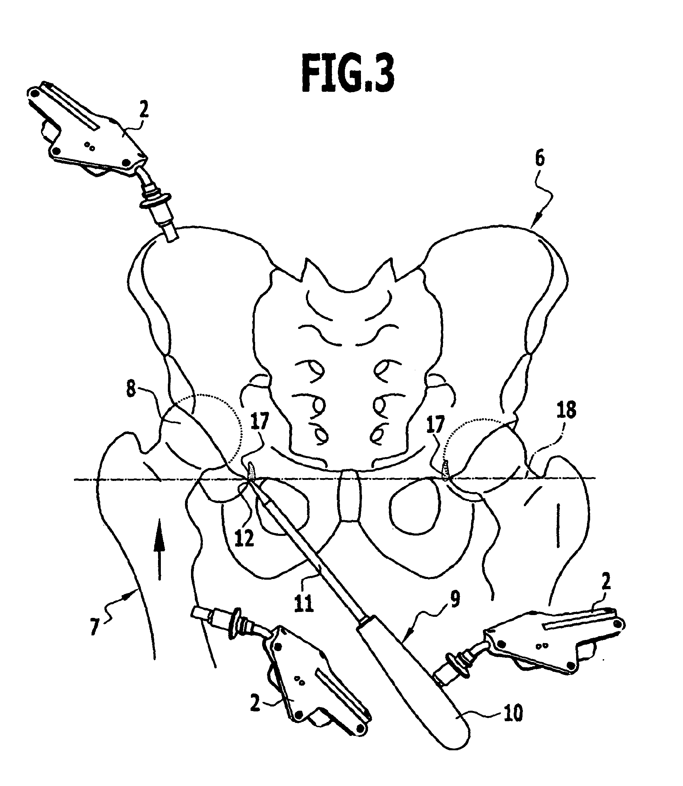

Des weiteren wird ein Tastinstrument 9 benutzt, das ebenfalls ein Markierelement 2 trägt und das im wesentlichen einen Handgriff 10 und einen daran gehaltenen, länglichen Schaft 11 mit einer Spitze 12 aufweist (Figur 3).

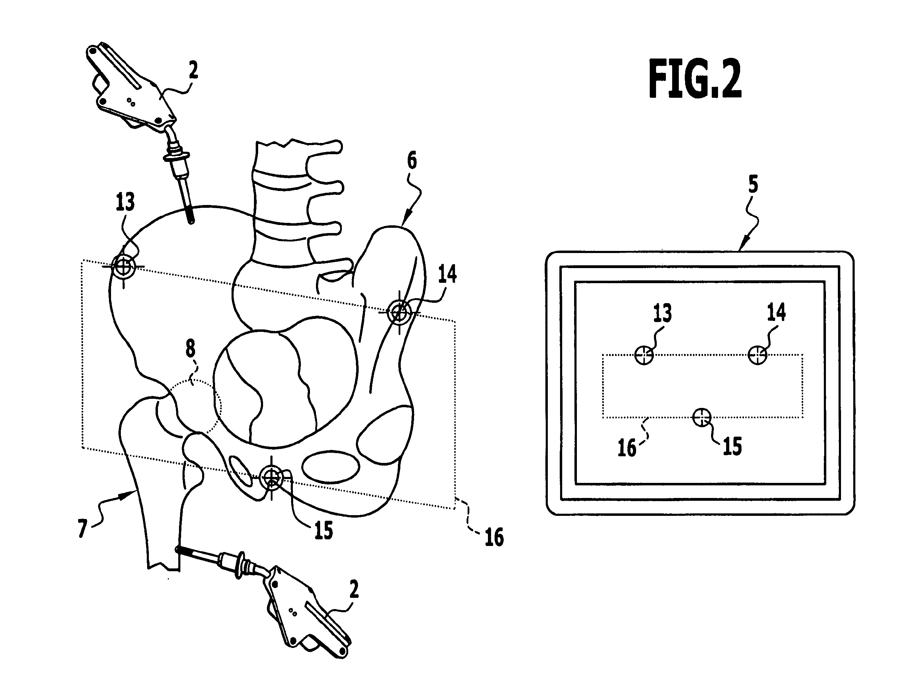

Der Operateur setzt das Tastinstrument 9 an verschiedenen markanten Punkten des Beckenknochens 6 so an, daß die Spitze 12 direkt auf diesen markanten Punkten aufsitzt. Dies ist durch die Haut hindurch möglich, die an diesen Stellen unmittelbar auf dem Beckenknochen aufliegt. Es handelt sich dabei um die beiden Spinae illiacae anteriores superiores 13, 14 an der Oberseite des Beckenknochens 6 und um die Symphysis pubica 15 an der Unterseite des Beckenknochens 6 und in dessen Mitte. Die Lagedaten dieser drei markanten Punkte werden über das navigierte Tastinstrument 9 der Datenverarbeitungseinrichtung 4 zugeführt, die aus diesen drei Punkten eine Ebene berechnet, nämlich die sogenannte Beckeneingangsebene 16 (Figur 2).

In einem nächsten Schritt wird mit Hilfe des Tastinstrumentes 9 an der Seite des Beckenknochens, an der das Implantat eingesetzt werden soll, ein kleiner Vorsprung in der Nähe des Hüftgelenkes ertastet, nämlich der sogenannte tear drop point 17 (Köhlersche Tränenfigur). Dieser Punkt ist relativ gut zu tasten und so klein, daß mit hoher Genauigkeit die Lagedaten dieses Punktes bestimmt und der Datenverarbeitungseinrichtung zugeführt werden können.

Die Datenverarbeitungseinrichtung berechnet eine Linie, die parallel zu der Verbindungslinie der beiden Spinae illiacae anteriores superiores verläuft und die durch den getasteten tear drop point 17 hindurchgeht. Die so erzeugte Linie wird tear drop Linie 18 genannt, sie verbindet die beiden tear drop points 17 auf gegenüberliegenden Seiten des Beckenknochens 6.

Die Datenverarbeitungseinrichtung 4 berechnet nunmehr aus den Lagedaten der Beckeneingangsebene 16 und den Lagedaten der tear drop Linie 18 eine tear drop Ebene 19, die senkrecht auf der Beckeneingangsebene 16 steht und in der die tear drop Linie 18 liegt. Während die Beckeneingangsebene 16 bei aufrechtem Beckenknochen 6 im wesentlichen eine senkrechte Frontalebene ist, verläuft die tear drop Ebene 19 im wesentlichen horizontal und geht durch die beiden tear drop points 17 hindurch.

In einem nächsten Schritt wird in dem Beckenknochen 6 eine Bohrung in medialer Richtung eingebracht, durch die der Beckenknochen 6 von außen nach innen durchbohrt wird. Diese Bohrung 20 verläuft im wesentlichen parallel zur tear drop Linie 18 und in einer Höhe über der tear drop Ebene 19, die etwa in der Mitte der vorgesehenen Aufnahmevertiefung 21 liegt. Diese Höhe ist aufgrund der verwendeten Werkzeuge abschätzbar, diese Werkzeuge sind in der Regel als halbkugelige Fräsköpfe ausgebildet, so daß durch die Auswahl eines bestimmten Fräskopfes auch die Größe der Aufnahmevertiefung festgelegt ist.

Es ist somit für den Operateur möglich, die Höhe der Bohrung 20 oberhalb der tear drop Ebene 19 in gewissen Grenzen abzuschätzen.

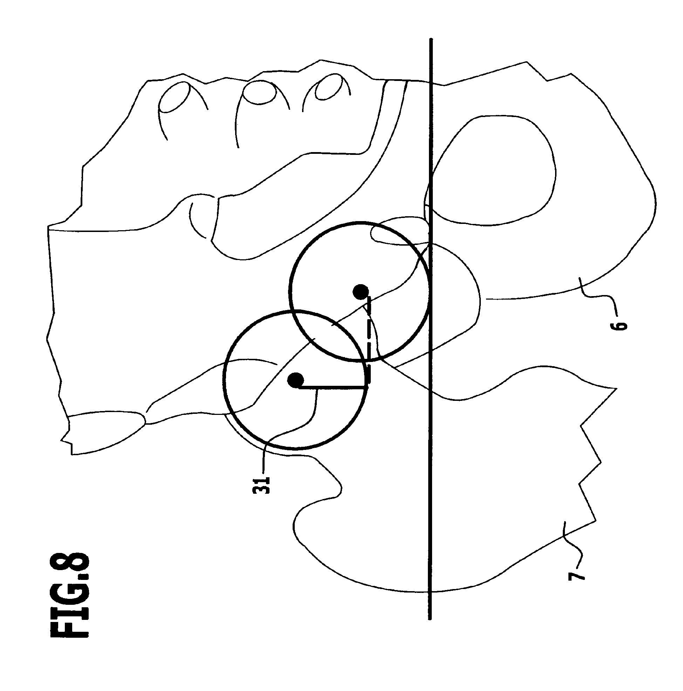

Bei der Abschätzung der Höhe der Bohrung 20 oberhalb der tear drop Ebene 19 ist gegebenenfalls auch die Lage des sekundären Acetabulums zu berücksichtigen, in dem der Femurknochen 7 vor der Operation gelagert ist. Wenn der Abstand des primären Acetabulums, in dem der Femurknochen nach der Operation gelagert werden soll, und des sekundären Acetabulums in cranialer Richtung einen bestimmten Wert überschreitet, kann dies zu einer Verlagerung des Femurknochens relativ zum Beckenknochen führen, bei der durch übermäßige Dehnung Schädigungen des Muskelgewebes und der Nerven auftreten könnten. Um dies zu vermeiden, ist gemäß einer bevorzugten Ausführungsform der Erfindung vorgesehen, daß die Lage des sekundären Acetabulums am Beckenknochen und insbesondere der Abstand zu der tear drop Ebene bestimmt werden. Dies kann mit Hilfe des Tasters 9 erfolgen, mit dem die Lagerfläche des sekundären Acetabulums punktweise erfaßt wird. Aus diesen Lagedaten und der Annahme, daß diese Lagerfläche annähernd kugelig ausgebildet ist, kann die Datenverarbeitungseinrichtung den Mittelpunkt des sekundären Acetabulums berechnen und daraus einen Abstand der Mittelpunkte des primären vom sekundären Acetabulum, diese Verschiebung ist in Figur 8 mit dem Bezugszeichen 31 gekennzeichnet.

Es kann vorgesehen sein, daß der Wert dieser Verschiebung einen Maximalwert nicht überschreiten darf, also beispielsweise einen Wert zwischen 25 und 40 mm, den der Operateur selbst vorgibt. Dadurch kann eine Korrektur der Lage des primären Acetabulums dahingehend erfolgen, daß die Höhe des primären Acetabulums über der tear drop Ebene größer wird, der Rand des primären Acetabulums also nicht mehr in die tear drop Ebene fällt, sondern darüber liegt.

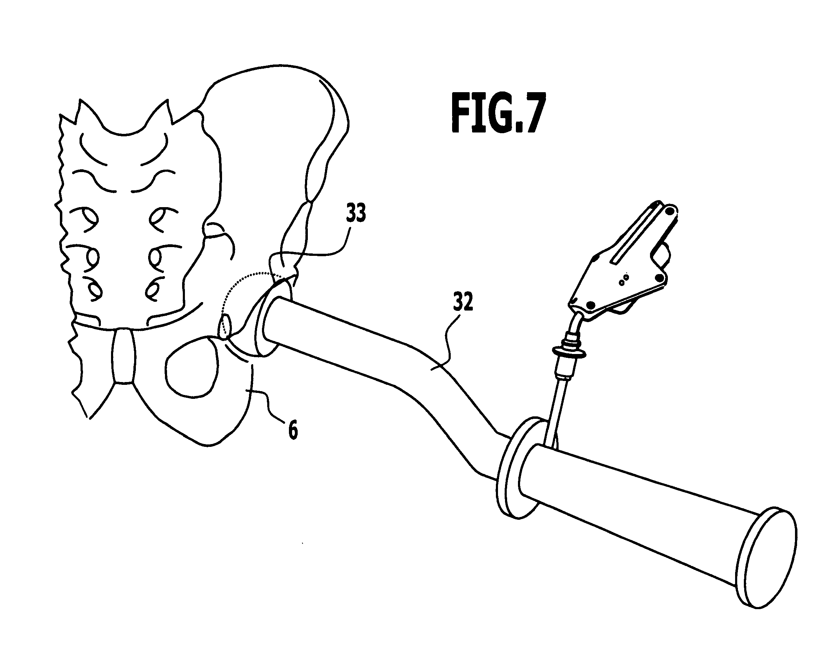

Die Lage des sekundären Acetabulums könnte auch mit einem navigierten Probeimplantat oder einem navigierten Taster mit einer kugeligen Anlagefläche bestimmt werden, wie dies in Figur 7 dargestellt ist. Ein solcher Taster 32 wird mit der kugeligen Anlagefläche 33 in das sekundäre Acetabulum eingelegt. Die Datenverarbeitungseinrichtung kann dann aus dem Verlauf der kugeligen Anlagefläche 33 deren Mittelpunkt bestimmen und die Lagedaten dieses Mittelpunktes als Mitte des sekundären Acetabulums für die Abstandsbestimmung vom primären Acetabulum verwenden. Die Bohrung 20 wird in anterior-posteriorer Richtung so angeordnet, daß sie ebenfalls im wesentlichen in der Mitte der zu erstellenden Aufnahmevertiefung verläuft. Auch dies läßt sich aus abschätzen, so daß der Operateur den Eintrittspunkt und die Richtung der Bohrung 20 bestimmen kann. Dies wird vorzugsweise mit Hilfe des Tastinstrumentes 9 unterstützt, der Operateur kann nämlich das Tastinstrument, das navigiert ist, in die entsprechende Lage bringen und dann, zum Beispiel durch leichte Hammerschläge, einen Eintrittspunkt für die Bohrung 20 markieren.

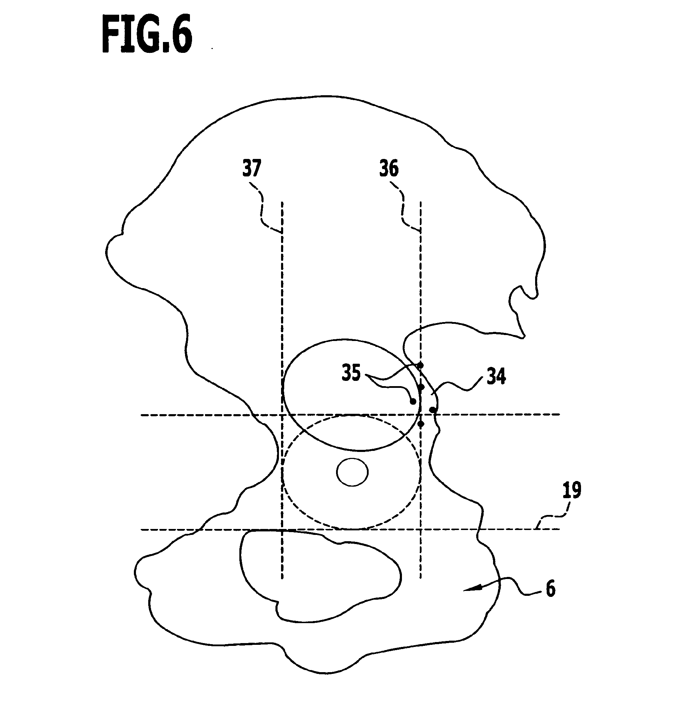

Die Aufnahmevertiefung sollte derart positioniert sein, daß der als Knochenvorsprung ausgebildete halbmondförmige dorsale Rand 34 (Figur 6) bei dem Einbringen der Aufnahmevertiefung nicht beschädigt wird. Um dies zu erreichen, kann vorgesehen werden, daß der Operateur mit dem Tastinstrument 9 einen oder mehrere Meßpunkte 35 am dorsalen Rand 34 abtastet und deren Lagedaten somit der Datenverarbeitungseinrichtung übermittelt. Diese Abtastung ist unter Umständen nicht sehr genau, da der dorsale Rand 34 eine gewisse Erstreckung hat und da es schwierig sein kann, den dorsalen Rand in seiner gesamten Erstreckung in der Operationswunde zu erreichen.

Die Datenverarbeitungseinrichtung kann daher so programmiert sein, daß sie beim Tasten einer bestimmten Anzahl von Meßpunkten 35 den Meßpunkt auswählt, der am meisten in ventraler Richtung verschoben ist.

Falls nur ein Meßpunkt bestimmt worden ist oder falls in der beschriebenen Weise aus einer Vielzahl der ventralste Meßpunkt ausgewählt worden ist, kann die Datenverarbeitungseinrichtung dann eine parallel zur Beckeneingangsebene verlaufende dorsale Frontalebene 36 durch diesen Meßpunkt legen und diese dorsale Frontalebene 36 zur Bestimmung der Lage der Aufnahmevertiefung in anterior-posterior-Richtung verwenden. Die Aufnahmevertiefung wird nämlich so plaziert, daß der Rand der Aufnahmevertiefung oder der Mittelpunkt von dieser dorsalen Frontalebene 36 einen Minimalwert nicht unterschreiten darf, dieser ist in jedem Fall so gewählt, daß die Aufnahmevertiefung den dorsalen Rand 34 nicht beschädigt.

Grundsätzlich wäre es möglich, auch am ventralen Rand des Beckenknochens 4 in gleicher Weise Meßpunkte zu bestimmen und dort eine ventrale Frontalebene 37 zu bestimmen, so daß der Bereich eingeengt wird, in dem sich die Aufnahmevertiefung erstrecken darf, dies kann auch dazu führen, daß die Größe der zur Herstellung der Aufnahmevertiefung verwendeten Werkzeuge und daher die Größe der implantierten Pfannen begrenzt wird.

Dem Operateur werden also durch die tear drop Ebene und gegebenenfalls die dorsale Frontalebene sowie eventuell eine ventrale Frontalebene geometrische Daten zur Verfügung gestellt, mit denen er die Positionierung der Aufnahmevertiefung exakt vorplanen und dann auch realisieren kann, korrigiert werden kann diese Anordnung noch durch die Randbedingung, nach der der Abstand zwischen sekundärem Acetabulum und primärem Acetabulum einen bestimmten Wert nicht überschreiten sollte.

Wenn die Daten in der beschriebenen Weise bestimmt sind, wird die Bohrung 20 mit einem in der Zeichnung nicht dargestellten Bohrgerät vorsichtig in den Beckenknochen eingebracht, um ein plötzliches Durchbrechen und damit eine Verletzung des Weichgewebes im Inneren des Beckenknochens zu verhindern.

Die Bohrung 20 wird dazu verwendet, die Dicke des Beckenknochens 6 im Bereich der Bohrung 20 zu bestimmen. Dazu wird durch die Bohrung 20 ein weiteres Tastinstrument 21 hindurchgesteckt, das ebenfalls ein Markierelement 2 trägt und damit navigierbar ist. Dieses ist ähnlich aufgebaut wie das Tastinstrument 9, gleiche Teile tragen daher die gleichen Bezugszeichen. Es ist lediglich die Spitze 12 rechtwinklig abgebogen, so daß sich an der Innenseite der abgebogenen Spitze 12 eine Anlagefläche 22 ausbildet, die beim Hindurchstecken des Tastinstrumentes 21 durch die Bohrung 20 und beim anschließenden Zurückziehen an der Rückseite 23 des Beckenknochens 6 anlegbar ist (Figur 4). Dadurch kann die Lage dieser Rückseite 23 bestimmt werden, die gleichzeitig ein Maß für die Dicke des Beckenknochens 6 ist.

Der auf diese Weise gewonnene Austrittspunkt der Bohrung 20 an der Rückseite 23 wird für das folgende Verfahren als Bezugspunkt 24 ausgewählt.

In einem leicht abgewandelten, nicht näher erläuterten Verfahren wird ein solcher Bezugspunkt 24' in etwas abgewandelter Weise dadurch gewonnen, daß mit einem navigierten Tastinstrument der Punkt angefahren wird, der oben als Eintrittspunkt der Bohrung 20 definiert worden ist. Ein solcher Punkt im Inneren des Beckenknochens 6 läßt sich beispielsweise dadurch gewinnen, daß das Tastinstrument 9 in medialer Richtung im Bereich der zu erstellenden Aufnahmevertiefung in derselben Lage wie die Bohrung 20 vorgeschoben wird, jedoch nicht ganz so weit, daß das Tastinstrument den Beckenknochen durchdringt.

In diesem Falle wird also der Bezugspunkt 24' im Inneren des Beckenknochens liegen.

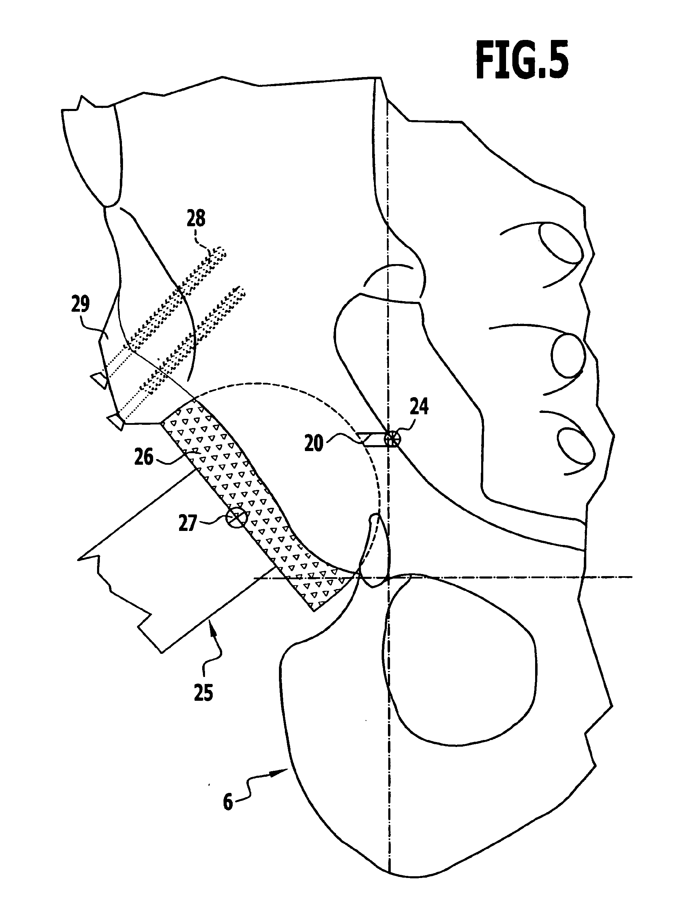

In beiden Fällen dient dieser Bezugspunkt 24 oder 24' dazu, dem Operateur ein Maß dafür zu geben, wie er ein Werkzeug 25, also beispielsweise den in Figur 5 dargestellten halbkugeligen Fräskopf, vorschieben muß, um in der gewünschten Lage und mit der gewünschten Tiefe eine Aufnahmevertiefung im Beckenknochen 6 zu erzeugen.

Die Datenverarbeitungseinrichtung erhält von dem Markierelement, das in den Beckenknochen 6 eingeschraubt ist, Informationen über die Lage des Beckenknochens und kann daraus die Lage des Bezugspunktes 24 beziehungsweise 24' berechnen, das navigierte Werkzeug 25 übermittelt seine Lagedaten über das mit ihm verbundene Markierelement ebenfalls der Datenverarbeitungseinrichtung, und somit kann auf dem Bildschirm 15 dargestellt werden, wie das Werkzeug 25 relativ zum Beckenknochen 6 vorgeschoben wird. Dazu können gegebenenfalls auch die tear drop Ebene 19 und / oder die tear drop Linie 18 in die Abbildung einkopiert werden.

Selbstverständlich können auch andere Daten auf dem Bildschirm angezeigt werden, beispielsweise die Lage des primären und des sekundären Acetabulums, die Lage der Meßpunkte 35, die Lage der dorsalen Frontalebene 36 oder gegebenenfalls einer ventralen Frontalebene 37 sowie die Daten des Abstandes 31 zwischen primärem und sekundärem Acetabulum in cranialer Richtung.

Der Operateur sieht beim Vorschieben des Werkzeuges 25, wie dies relativ zu den genannten Ebenen und damit zum Beckenknochen orientiert ist und wie tief das Werkzeug in den Beckenknochen eintritt.

Die Datenverarbeitungseinrichtung kann zusätzlich den Abstand der Außenfläche 26 des Werkzeuges 25 oder des Mittelpunktes 27 des Werkzeuges 25 von den Bezugspunkten 24 oder 24' bestimmen und erhält damit ein Maß für die Eindringtiefe des Werkzeuges 25. An Stelle einer direkten Abstandsbestimmung relativ zu den Bezugspunkten 24, 24' kann die Datenverarbeitungseinrichtung auch eine Sagittalebene bestimmen, die durch den Bezugspunkt 24 oder den Bezugspunkt 24' hindurchgeht. Es handelt sich dabei im wesentlichen um eine Ebene, die sowohl senkrecht auf der Beckeneingangsebene als auch auf der tear drop Ebene steht.

In jedem Falle wird die Datenverarbeitungseinrichtung überwachen, daß die Grenzwerte zwischen dem Werkzeug (Außenfläche oder Mittelpunkt) und den geometrischen Ebenen oder Punkten, die in der vorstehenden Weise bestimmt worden sind, eingehalten werden. Sobald eine Abweichung von den Extremwerten auftritt, wird die Datenverarbeitungseinrichtung eine Warnung abgeben und somit verhindern, daß der Operateur das Werkzeug in unerwünschter Weise führt, also beispielsweise zu tief in den Beckenknochen eintreibt, zu dicht am dorsalen Rand 34 arbeitet oder einen zu großen Abstand von dem sekundären Acetabulum anstrebt.

Bei der Verlagerung der knöchernen Vertiefung in caudaler Richtung ist es unter Umständen notwendig, einen Teil der natürlichen Vertiefung des sekundären Acetabulums mit einem Knochenimplantat auszufüllen, um die Möglichkeit zu erhalten, in einer tiefer gelegenen Position eine vollständig im knöchernen Material angeordnete Aufnahmevertiefung erzeugen zu können. Dies wird üblicherweise durch das Einsetzen von Knochenimplantaten in dem Bereich der ursprünglichen natürlichen Aufnahmevertiefung erreicht, in Figur 5 ist dies angedeutet durch ein mittels Knochenschrauben 28 am Beckenknochen 6 gehaltenes Knochenimplantat 29. Dies kann beispielsweise aus dem entsprechend bearbeiteten und resezierten Femurkopf gebildet werden.

## Patentansprüche

1. Chirurgisches Navigationssystem für ein navigiertes Werkzeug (25) zur Herstellung einer Aufnahmevertiefung für eine Hüftgelenkpfanne in einem navigierten Beckenknochen (6) mit einem navigierten Tastinstrument (9) und mit einer Datenverarbeitungseinrichtung (4) zur Bearbeitung der Lagedaten der navigierten Teile, **dadurch gekennzeichnet, dass** die Datenverarbeitungseinrichtung (4) derart programmiert ist, dass sie aus den mit Hilfe des navigierten Tastinstrumentes (9) bestimmten Lagedaten eines tear drop points (17) (Köhlersche Tränenfigur) am Beckenknochen (6), aus den Lagedaten der Beckeneingangsebene (16) und aus den Lagedaten der beiden Spinae illiacae anteriores superiores (13, 14) des Beckenknochens (6) eine senkrecht auf der Beckeneingangsebene (16) stehende, parallel zu einer die beiden Spinae illiacae anteriores superiores (13, 14) verbindenden Linie und durch den getasteten tear drop point (17) verlaufende tear drop Ebene (19) bestimmt, dass sie in einer vorgegebenen Höhe oberhalb der tear drop Ebene (19) in einer vorgegebenen Position in anterior-posteriorer Richtung und in einem vorgegebenen Abstand von der Außenfläche des Beckenknochens (6) in lateral-medialer Richtung einen Bezugspunkt (24, 24') bestimmt, dass sie unter Verwendung der Lagedaten eines gegenüber dem primären Acetabulum in kranialer Richtung verschobenen sekundären Acetabulums die Höhe des Bezugspunktes (24, 24') oberhalb der tear drop Ebene (19) derart berechnet, dass ein maximaler Wert des Abstandes zwischen der Höhe des Bezugspunktes (24, 24') und dem sekundären Acetabulum nicht überschritten wird, und dass sie die Lage des Werkzeuges (25) relativ zu dem Beckenknochen (6) und relativ zu dem Bezugspunkt (24, 24') berechnet.

2. Navigationssystem nach Anspruch 1, **dadurch gekennzeichnet, dass** die Datenverarbeitungseinrichtung (4) derart programmiert ist, dass sie aus den mittels des Tastinstrumentes (9) bestimmten Lagedaten der beiden Spinae illiacae anteriores superiores (13, 14) und der Symphysis pubica (15) des Beckenknochens (6) die Beckeneingangsebene (16) berechnet.

3. Navigationssystem nach einem der Ansprüche 1 oder 2, **dadurch gekennzeichnet, dass** die Datenverarbeitungseinrichtung (4) derart programmiert ist, dass sie die Höhe des Bezugspunktes (24, 24') oberhalb der tear drop Ebene (19) in Abhängigkeit von der Dimensionierung des Werkzeuges (25) und damit der gewünschten Größe der Aufnahmevertiefung so berechnet, dass der untere Rand der Aufnahmevertiefung etwa in der tear drop Ebene (19) liegt.

4. Navigationssystem nach einem der voranstehenden Ansprüche, **dadurch gekennzeichnet, dass** der maximale Wert bei 40 mm liegt.

5. Navigationssystem nach einem der Ansprüche 1 bis 3, **dadurch gekennzeichnet, dass** der maximale Wert bei 25 mm liegt.

6. Navigationssystem nach einem der voranstehenden Ansprüche, **dadurch gekennzeichnet, dass** die Datenverarbeitungseinrichtung (4) derart programmiert ist, dass sie den maximalen Wert zwischen einem zentralen Punkt des sekundären Acetabulums und den Bezugspunkten (24, 24') bestimmt.

7. Navigationssystem nach Anspruch 6, **dadurch gekennzeichnet, dass** die Datenverarbeitungseinrichtung (4) derart programmiert ist, dass sie aus den Lagedaten eines navigierten, gegenüber dem navigierten Beckenknochen (6) in dem sekundären Acetabulum präoperativ bewegten Femurknochen (7) den Mittelpunkt der Bewegung des Femurknochens (7) bestimmt und diesen Mittelpunkt als zentralen Punkt des sekundären Acetabulums verwendet.

8. Navigationssystem nach Anspruch 6, **dadurch gekennzeichnet, dass** die Datenverarbeitungseinrichtung (4) derart programmiert ist, dass sie aus den Lagedaten eines navigierten Tastinstrumentes (32), welches an die Lagerfläche des sekundären Acetabulums angelegt ist, die Lage des sekundären Acetabulums bestimmt und dass sie aus den Lagedaten des sekundären Acetabulums deren Mittelpunkt als zentralen Punkt des sekundären Acetabulums bestimmt.

9. Navigationssystem nach einem der voranstehenden Ansprüche, **dadurch gekennzeichnet, dass** die Datenverarbeitungseinrichtung (4) derart programmiert ist, dass sie die Position des Bezugspunktes (24, 24') in anterior-posteriorer Richtung derart berechnet, dass er etwa in der Mitte der Aufnahmevertiefung liegt.

10. Navigationssystem nach einem der voranstehenden Ansprüche, **dadurch gekennzeichnet, dass** die Datenverarbeitungseinrichtung (4) derart programmiert ist, dass sie aus Lagedaten, die durch Anlage eines navigierten Tasters (9) an einen oder mehrere Messpunkte (35) des dorsalen Randes (34) des Beckenknochens (6) im Bereich des primären Acetabulums bestimmt worden sind, eine Position der Aufnahmevertiefung derart bestimmt, dass der Abstand des Randes der Aufnahmevertiefung von dem Messpunkt oder den Messpunkten (35) des dorsalen Randes (34) einen bestimmten Minimalwert nicht unterschreitet.

11. Navigationssystem nach Anspruch 10, **dadurch gekennzeichnet, dass** die Datenverarbeitungseinrichtung (4) derart programmiert ist, dass sie aus den Lagedaten von mehreren Messpunkten (35) des dorsalen Randes (34) des Beckenknochens (6) denjenigen auswählt, der am weitesten in ventraler Richtung angeordnet ist und dass sie den bestimmten Minimalwert aus den Lagedaten dieses ausgewählten Messpunktes (35) bestimmt.

12. Navigationssystem nach einem der Ansprüche 10 oder 11, **dadurch gekennzeichnet, dass** die Datenverarbeitungseinrichtung (4) derart programmiert ist, dass sie eine durch einen Messpunkt (35) des dorsalen Randes (34) hindurchgehende dorsale Frontalebene (36) bestimmt und den bestimmten Minimalwert zwischen dem Rand der Aufnahmevertiefung und dieser dorsalen Frontalebene (36) bestimmt.

13. Navigationssystem nach einem der voranstehenden Ansprüche, **dadurch gekennzeichnet, dass** die Datenverarbeitungseinrichtung (4) derart programmiert ist, dass sie den Abstand des Bezugspunktes (24) von der Außenfläche des Beckenknochens (6) aus den Lagedaten eines navigierten Tastinstrumentes (21) bestimmt, welches durch eine künstliche Öffnung (20) im Bereich des primären Acetabulums des Beckenknochens (6) in medialer Richtung durch den Beckenknochen (6) bis zur Rückseite (23) des Beckenknochens (6) hindurchgesteckt wird.

14. Navigationssystem nach Anspruch 13, **dadurch gekennzeichnet, dass** das Tastinstrument (21) zur Bestimmung des Abstandes des Bezugspunktes (24) von der Außenfläche des Beckenknochens (6) eine seitlich von einem Schaft (11) abstehende Anlagefläche (12) zur Anlage an der Rückseite (23) des Beckenknochens (6) aufweist.

15. Navigationssystem nach einem der voranstehenden Ansprüche, **dadurch gekennzeichnet, dass** die Datenverarbeitungseinrichtung (4) derart programmiert ist, dass sie den kleinsten Abstand der der Knochenbearbeitung dienenden Außenfläche (26) des Werkzeuges (25) von dem Bezugspunkt (24, 24') berechnet.

16. Navigationssystem nach einem der Ansprüche 1 bis 14, **dadurch gekennzeichnet, dass** die Datenverarbeitungseinrichtung (4) derart programmiert ist, dass sie den kleinsten Abstand des Mittelpunktes (27) des Werkzeuges (25) von den Bezugspunkten (24, 24') berechnet.

17. Navigationssystem nach einem der Ansprüche 1 bis 14, **dadurch gekennzeichnet, dass** die Datenverarbeitungseinrichtung (4) derart programmiert ist, dass sie den kleinsten Abstand der der Knochenbearbeitung dienenden Außenfläche (26) des Werkzeuges (25) von einer durch den Bezugspunkt (24, 24') hindurchgehenden Sagittalebene berechnet.

18. Navigationssystem nach einem der Ansprüche 1 bis 14, **dadurch gekennzeichnet, dass** die Datenverarbeitungseinrichtung (4) derart programmiert ist, dass sie den kleinsten Abstand des Mittelpunktes (27) des Werkzeuges (25) von einer durch den Bezugspunkt (24, 24') hindurchgehenden Sagittalebene berechnet.

19. Navigationssystem nach einem der Ansprüche 14 bis 18, **dadurch gekennzeichnet, dass** die Datenverarbeitungseinrichtung (4) derart programmiert ist, dass sie die relative Lage des Werkzeuges (25) und des Bezugspunktes (24, 24') oder der durch den Bezugspunkt (24, 24') gegebenen Sagittalebene auf einer Anzeige (5) anzeigt.

20. Navigationssystem nach einem der Ansprüche 7 bis 19, **dadurch gekennzeichnet, dass** die Datenverarbeitungseinrichtung (4) derart programmiert ist, dass sie die relative Lage des Werkzeuges (25) und des sekundären Acetabulums auf einer Anzeige (5) anzeigt.

21. Navigationssystem nach einem der Ansprüche 10 bis 19, **dadurch gekennzeichnet, dass** die Datenverarbeitungseinrichtung (4) derart programmiert ist, dass sie die relative Lage des Werkzeuges (25) und der Messpunkte (35) des dorsalen Randes (34) des Beckenknochens (6) und / oder der dorsalen Frontalebene (36) auf einer Anzeige (5) anzeigt.

22. Navigationssystem nach einem der voranstehenden Ansprüche, **dadurch gekennzeichnet, dass** die Datenverarbeitungseinrichtung (4) derart programmiert ist, dass sie ein Warnsignal erzeugt, sobald kritische Werte über- beziehungsweise unterschritten werden.

## Claims

1. Surgical navigation system for a navigated tool (25) for producing a cavity to receive an acetabulum in a navigated pelvic bone (6) with a navigated scanning instrument (9) and with a data processing unit (4) for processing the position data of the navigated parts, **characterised in that** the data processing unit (4) is programmed in such a way that from the position data of a tear-drop point (17) (Köhler's area) on the pelvic bone (6) determined by means of the navigated scanning instrument (9), from the position data of the plane of the pelvic inlet (16) and from the position data of the two anterior superior iliac spines (13, 14) of the pelvic bone (6), the data processing unit (4) determines a tear-drop plane (19), which lies perpendicular to the plane of the pelvic inlet (16) and extends parallel to a line connecting the two anterior superior iliac spines (13, 14) and through the scanned tear-drop point (17), **in that** the data processing unit (4) determines a reference point (24, 24') at a defined height above the tear-drop plane (19) and in a defined position in the anterior-posterior direction and at a defined spacing from the outer surface of the pelvic bone (6) in the lateral-medial direction, **in that** from position data of a secondary acetablum displaced in the cranial direction relative to the primary acetabulum, the data processing unit (4) calculates the height of the reference point (24, 24') above the tear-drop plane (19) in such a manner that a maximum value of the spacing between the height of the reference point (24, 24') and the secondary acetabulum is not exceeded, and **in that** the data processing unit (4) calculates the position of the tool (25) relative to the pelvic bone (6) and relative to the reference point (24, 24').

2. Navigation system according to claim 1, **characterised in that** the data processing unit (4) is programmed in such a way that it calculates the plane of the pelvic inlet (16) from the position data of the two anterior superior iliac spines (13, 14) and the pubic symphysis (15) of the pelvic bone (6) determined by means of the scanning instrument (9).

3. Navigation system according to one of claims 1 or 2, **characterised in that** the data processing unit (4) is programmed in such a way that it calculates the height of the reference point (24, 24') above the tear-drop plane (19) as a function of the dimensioning of the tool (25) and thus of the desired size of the receiving cavity such that the lower edge of the receiving cavity lies approximately in the tear-drop plane (19).

4. Navigation system according to one of the preceding claims, **characterised in that** the maximum value amounts to 40 mm.

5. Navigation system according to one of claims 1 to 3, **characterised in that** the maximum value amounts to 25 mm.

6. Navigation system according to one of the preceding claims, **characterised in that** the data processing unit (4) is programmed in such a way that it determines the maximum value between a central point of the secondary acetabulum and the reference points (24, 24').

7. Navigation system according to claim 6, **characterised in that** the data processing unit (4) is programmed in such a way that from the position data of a navigated femur bone moved pre-operatively in the secondary acetabulum relative to the navigated pelvic bone (6), it determines the mid-point of the movement of the femur bone (7) and uses this mid-point as central point of the secondary acetabulum.

8. Navigation system according to claim 6, **characterised in that** the data processing unit (4) is programmed in such a way that it determines the disposition of the secondary acetabulum from the position data of a navigated scanning instrument (32), which is placed against the supporting surface of the secondary acetabulum, and that from the position data of the secondary acetabulum it determines the mid-point thereof as central point of the secondary acetabulum.

9. Navigation system according to one of the preceding claims, **characterised in that** the data processing unit (4) is programmed in such a way that it calculates the position of the reference point (24, 24') in the anterior-posterior direction such that it lies approximately in the centre of the receiving cavity.

10. Navigation system according to one of the preceding claims, **characterised in that** the data processing unit (4) is programmed in such a way that from position data, which have been determined by placing a navigated probe (9) against one or more measurement points (35) of the dorsal edge (34) of the pelvic bone (6) in the region of the primary acetabulum, it determines a position of the receiving cavity such that the spacing of the edge of the receiving cavity from the measurement point or the measurement points (35) of the dorsal edge (34) does not fall below a defined minimum value.

11. Navigation system according to claim 10, **characterised in that** the data processing unit (4) is programmed in such a way that from the position data of a plurality of measurement points (35) of the dorsal edge (34) of the pelvic bone (6) it selects the one arranged furthest in the ventral direction, and that it determines the defined minimum value from the position data of this selected measurement point (35).

12. Navigation system according to one of claims 10 or 11, **characterised in that** the data processing unit (4) is programmed in such a way that it determines a dorsal frontal plane (36) extending through a measurement point (35) of the dorsal edge (34) and determines the defined minimum value between the edge of the receiving cavity and this dorsal frontal plane (36).

13. Navigation system according to one of the preceding claims, **characterised in that** the data processing unit (4) is programmed in such a way that it determines the spacing of the reference point (24) from the outer surface of the pelvic bone (6) from the position data of a navigated scanning instrument (21), which is inserted through an artificial opening (20) in the region of the primary acetabulum of the pelvic bone (6) in the medial direction through the pelvic bone (6) as far as the rear side (23) of the pelvic bone (6).

14. Navigation system according to claim 13, **characterised in that** to determine the spacing of the reference point (24) from the outer surface of the pelvic bone (6) the scanning instrument (21) has an abutment surface (12) protruding laterally from a shaft (11) for placement against the rear side (23) of the pelvic bone (6).

15. Navigation system according to one of the preceding claims, **characterised in that** the data processing unit (4) is programmed in such a way that it calculates the shortest spacing of the outer surface (26) of the tool (25) serving to machine bone from the reference point (24, 24').

16. Navigation system according to one of claims 1 to 14, **characterised in that** the data processing unit (4) is programmed in such a way that it calculates the shortest spacing of the central point (27) of the tool (25) from the reference points (24, 24').

17. Navigation system according to one of claims 1 to 14, **characterised in that** the data processing unit (4) is programmed in such a way that it calculates the shortest spacing of the outer surface (26) of the tool (25) serving to machine bone from a sagittal plane extending through the reference point (24, 24').

18. Navigation system according to one of claims 1 to 14, **characterised in that** the data processing unit (4) is programmed in such a way that it calculates the shortest spacing of the central point (27) of the tool (25) from a sagittal plane extending through the reference point (24, 24').

19. Navigation system according to one of claims 14 to 18, **characterised in that** the data processing unit (4) is programmed in such a way that it displays the relative disposition of the tool (25) and the reference point (24, 24') or the sagittal plane defined by the reference point (24, 24') on a display (5).

20. Navigation system according to one of claims 7 to 19, **characterised in that** the data processing unit (4) is programmed in such a way that it displays the relative disposition of the tool (25) and the secondary acetabulum on a display (5).

21. Navigation system according to one of claims 10 to 19, **characterised in that** the data processing unit (4) is programmed in such a way that it displays the relative disposition of the tool (25) and the measurement points (35) of the dorsal edge (34) of the pelvic bone (6) and/or the dorsal frontal plane (36) on a display (5).

22. Navigation system according to one of the preceding claims, **characterised in that** the data processing unit (4) is programmed in such a way that it generates a warning signal as soon as critical values are exceeded or fallen below.

## Revendications

1. Système de navigation chirurgical pour un outil (25) navigué, en vue de la réalisation d'un évidement de réception pour une cupule d'articulation de la hanche dans un os de bassin (6) navigué, comprenant un instrument palpeur (9) navigué et un dispositif de traitement de données (4) pour le traitement de données de localisation des parties naviguées, **caractérisé en ce que** le dispositif de traitement de données (4) est programmé de façon
à déterminer, à partir des données de positionnement d'un point tear drop ou point en goutte de larme (17) (figure en larme de Kohler) sur l'os de bassin (6) déterminées à l'aide de l'instrument palpeur (9) navigué, à partir des données de positionnement du plan d'entrée de bassin (16) et à partir des données de positionnement des deux épines iliaques antéro-supérieures (13, 14) de l'os de bassin (6), un plan tear drop ou plan de goutte de larme (19) orienté perpendiculairement au plan d'entrée de bassin (16), parallèle à une ligne reliant les deux épines iliaques antéro-supérieures (13, 14), et passant par le point en goutte de larme (17) ayant été palpé,
à déterminer un point de référence (24, 24'), à une hauteur prédéterminée au-dessus du plan de goutte de larme (19), dans une position prédéterminée en direction antéropostérieure et à une distance prédéterminée de la surface extérieure de l'os de bassin (6) en direction latérale-médiale,
à calculer, en utilisant les données de positionnement d'un acetabulum secondaire décalé en direction craniale par rapport à l'acetabulum primaire, la hauteur du point de référence (24, 24') au-dessus du plan de goutte de larme (19) de manière à ne pas dépasser une valeur maximale de la distance entre la hauteur du point de référence (24, 24') et l'acetabulum secondaire, et
à calculer le positionnement de l'outil (25) par rapport à l'os de bassin (6) et par rapport au point de référence (24, 24').

2. Système de navigation selon la revendication 1, **caractérisé en ce que** dispositif de traitement de données (4) est programmé de façon
à calculer le plan d'entrée de bassin (16), à partir des données de positionnement des deux épines iliaques antéro-supérieures (13, 14) et de la symphyse pubienne (15) de l'os de bassin (6), qui ont été déterminées au moyen de l'instrument palpeur (9).

3. Système de navigation selon l'une des revendications 1 ou 2, **caractérisé en ce que** le dispositif de traitement de données (4) est programmé de façon à calculer la hauteur du point de référence (24, 24') au-dessus du plan de goutte de larme (19) en fonction du dimensionnement de l'outil (25) et ainsi de la grandeur souhaitée de l'évidement de réception, de manière à ce que le bord inférieur de l'évidement de réception se situe environ dans le plan de goutte de larme (19).

4. Système de navigation selon l'une des revendications précédentes, **caractérisé en ce que** ladite valeur maximale se situe à 40 mm.

5. Système de navigation selon l'une des revendications 1 à 3, **caractérisé en ce que** la dite valeur maximale se situe à 25 mm.

6. Système de navigation selon l'une des revendications précédentes, **caractérisé en ce que** le dispositif de traitement de données (4) est programmé de façon à déterminer la valeur maximale entre un point central de l'acetabulum secondaire et les points de référence (24, 24').

7. Système de navigation selon la revendication 6, **caractérisé en ce que** le dispositif de traitement de données (4) est programmé de façon
à déterminer, à partir des données de positionnement d'un os de fémur (7) navigué, déplacé de manière préopératoire dans l'acetabulum secondaire par rapport à l'os de bassin (6) navigué, le centre du mouvement de l'os de fémur (7), et
à utiliser ce centre en tant que point central de l'acetabulum secondaire.

8. Système de navigation selon la revendication 6, **caractérisé en ce que** dispositif de traitement de données (4) est programmé de façon
à déterminer, à partir des données de positionnement d'un instrument palpeur (32), qui est appliqué contre la surface de palier de l'acetabulum secondaire, la position de l'acetabulum secondaire, et
à déterminer, à partir des données de positionnement de l'acetabulum secondaire, le centre de celui-ci en tant que point central de l'acetabulum secondaire.

9. Système de navigation selon l'une des revendications précédentes, **caractérisé en ce que** le dispositif de traitement de données (4) est programmé de façon à calculer la position du point de référence (24, 24') dans la direction antéropostérieure de manière à ce qu'il se situe environ au milieu de l'évidement de réception.

10. Système de navigation selon l'une des revendications précédentes, **caractérisé en ce que** le dispositif de traitement de données (4) est programmé de façon à déterminer, à partir des données de positionnement, qui ont été déterminées par l'application d'un palpeur (9) navigué contre un ou plusieurs points de mesure (35) du bord dorsal (34) de l'os de bassin (6) dans la région de l'acetabulum primaire, une position de l'évidement de réception, de manière telle que la distance du bord de l'évidement de réception au point de mesure ou aux points de mesure (35) du bord dorsal (34) ne passe pas en-dessous d'une valeur minimale définie.

11. Système de navigation selon la revendication 10, **caractérisé en ce que** le dispositif de traitement de données (4) est programmé de façon
à sélectionner, à partir des données de positionnement de plusieurs points de mesure (35) du bord dorsal (34) de l'os de bassin (6), celui qui est agencé le plus loin en direction ventrale, et
à déterminer ladite valeur minimale définie, à partir des données de positionnement de ce point de mesure (35) sélectionné.

12. Système de navigation selon l'une des revendications 10 ou 11, **caractérisé en ce que** le dispositif de traitement de données (4) est programmé de façon à déterminer un plan frontal dorsal (36) passant par un point de mesure (35) du bord dorsal (34), et
à déterminer ladite valeur minimale définie, entre le bord de l'évidement de réception et ce plan frontal dorsal (36).

13. Système de navigation selon l'une des revendications précédentes, **caractérisé en ce que** le dispositif de traitement de données (4) est programmé de façon à déterminer la distance du point de référence (24) à la surface extérieure de l'os de bassin (6), à partir des données de positionnement d'un instrument palpeur (21) navigué, que l'on introduit à travers une ouverture artificielle (20) dans la région de l'acetabulum primaire de l'os de bassin (6), en direction médiale, à travers l'os de bassin (6), jusqu'au côté arrière (23) de l'os de bassin (6).

14. Système de navigation selon la revendication 13, **caractérisé en ce que** l'instrument palpeur (21) pour la détermination de la distance du point de référence (24) à la surface extérieure de l'os de bassin (6), présente une surface d'appui (12) faisant saillie latéralement d'un corps allongé (11) et destinée à venir s'appuyer contre le côté arrière (23) de l'os de bassin (6).

15. Système de navigation selon l'une des revendications précédentes, **caractérisé en ce que** le dispositif de traitement de données (4) est programmé de façon à calculer la distance la plus petite de la surface extérieure (26) de l'outil (25), qui sert à usiner l'os, au point de référence (24, 24').

16. Système de navigation selon l'une des revendications 1 à 14, **caractérisé en ce que** le dispositif de traitement de données (4) est programmé de façon à calculer la distance la plus petite du centre (27) de l'outil (25) aux points de référence (24, 24').

17. Système de navigation selon l'une des revendications 1 à 14, **caractérisé en ce que** le dispositif de traitement de données (4) est programmé de façon à calculer la distance la plus petite de la surface extérieure (26) de l'outil (25), qui sert à usiner l'os, à un plan sagittal passant par le point de référence (24, 24').

18. Système de navigation selon l'une des revendications 1 à 14, **caractérisé en ce que** le dispositif de traitement de données (4) est programmé de façon à calculer la distance la plus petite du centre (27) de l'outil (25) à un plan sagittal passant par le point de référence (24, 24').

19. Système de navigation selon l'une des revendications 14 à 18, **caractérisé en ce que** le dispositif de traitement de données (4) est programmé de façon à visualiser sur un dispositif de visualisation (5), le positionnement relatif de l'outil (25) et du point de référence (24, 24') ou du plan sagittal passant par le point de référence (24, 24').

20. Système de navigation selon l'une des revendications 7 à 19, **caractérisé en ce que** le dispositif de traitement de données (4) est programmé de façon à visualiser sur un dispositif de visualisation (5), le positionnement relatif de l'outil (25) et de l'acetabulum secondaire.

21. Système de navigation selon l'une des revendications 10 à 19, **caractérisé en ce que** le dispositif de traitement de données (4) est programmé de façon à visualiser sur un dispositif de visualisation (5), le positionnement relatif de l'outil (25) et des points de mesure (35) du bord dorsal (34) de l'os de bassin (6) et/ou du plan frontal dorsal (36).

22. Système de navigation selon l'une des revendications précédentes, **caractérisé en ce que** le dispositif de traitement de données (4) est programmé de façon à engendrer un signal d'alerte dès que l'on passe au-dessus, respectivement en-dessous de valeurs critiques.
